(19) Europäisches Patentamt
European Patent Office
Office européen des brevets

(11) EP 0 813 529 B1

(12) **FASCICULE DE BREVET EUROPEEN**

(45) Date de publication et mention de la délivrance du brevet:
**11.09.2002 Bulletin 2002/37**

(21) Numéro de dépôt: **96905907.0**

(22) Date de dépôt: **04.03.1996**

(51) Int Cl.[7]: **C07D 295/088**, C07C 279/36, C07D 521/00, C07C 279/14, A61K 31/155, A61K 31/435, A61K 31/535, A61K 31/415

(86) Numéro de dépôt international:
**PCT/FR96/00337**

(87) Numéro de publication internationale:
**WO 96/027593 (12.09.1996 Gazette 1996/41)**

(54) **ANALOGUES DE L'ARGININE AYANT UNE ACTIVITE EN TANT QU'INHIBITEURS DE LA NO SYNTHASE**

ARGINNIN ANALOGE MIT NO- SYNTAX INHIBIERENDERWIRKUNG

ARGININE ANALOGUES HAVING NITRIC OXIDE SYNTHASE INHIBITOR ACTIVITY

(84) Etats contractants désignés:
**AT BE CH DE DK ES FI FR GB IE IT LI NL SE**
Etats d'extension désignés:
**LT LV SI**

(30) Priorité: **04.03.1995 GB 9504350**

(43) Date de publication de la demande:
**29.12.1997 Bulletin 1997/52**

(73) Titulaire: **SOCIETE DE CONSEILS DE RECHERCHES ET D'APPLICATIONS SCIENTIFIQUES (S.C.R.A.S.)**
**75016 Paris (FR)**

(72) Inventeurs:
- **BROQUET, Colette**
  **F-92100 Boulogne (FR)**
- **CHABRIER DE LASSAUNIERE, Pierre-Etienne**
  **F-75016 Paris (FR)**

(74) Mandataire: **Bourgouin, André**
**BEAUFOUR IPSEN (S.C.A.F.)**
**42, rue du Docteur Blanche**
**75016 Paris (FR)**

(56) Documents cités:
**WO-A-91/04024**
**WO-A-93/24126**
**FR-A- 2 656 220**
**GB-A- 2 263 111**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne des dérivés de la L-arginine, un procédé pour leur préparation et des compositions pharmaceutiques les contenant.

Les composés de l'invention présente une activité biologique en tant qu'inhibiteurs de la NO synthase. Compte tenu du rôle potentiel de la NO synthase dans la physiopathologie (S. Moncada, R.M.J. Palmer et al., Nitric oxide: physiology, pathophysiology and pharmacology, Pharmaceutical reviews 43, 2, 109-142), de tels composés peuvent donc être intéressants comme agent hypotenseur, antibactérien, immunosuppresseur, antiartérosclérotique, vasotropique, analgésique, antimigraineux, ophtalmologique ou antidiabétique. Ainsi on peut envisager d'utiliser ces composés comme principe actif d'un médicament pour le traitement de pathologies du système nerveux central et périphérique telles que : infarctus cérébral ; migraine et céphalées ; épilepsy ; traumatismes cérébral ou de la moelle ; maladies neurodégénératives et/ou autoimmunes comme la maladie d'Alzheimer, la maladie de Parkinson, chorée d'Hunlington, sclérose latérale amyotrophique ; neuropathies cérébrales infectieuses (SIDA) ; douleur aiguë et chronique ; tolérance et dépendance au morphinique, psychotrope et à l'alcool ; neuropathie occulaire; dépression. On peut également envisager de les utiliser pour d'autres pathologies telles que celles liées à des dysfonctionnements du système gastrointestinal et urinaire de type inflammatoire ou non comme les colites ulcéreuses, gastrites, maladie de Crhonn, troubles des mictions, réflux gastro oesophagien, diarrhée, mais aussi pour des pathologies liées au système cardiovasculaire ou bronchopulmonaire comme hypotension, hypertension, atherosclerose, fibroses pulmonaires, sclérodermie, asthme, hypertension pulmonaire, cyrrhose, diabète, pour des maladies inflammatoires ou infectieuses comme arthrose, polyarthrites, choc septique, vasculite ou bien pour des troubles de l'érection, le priapisme ou contraception.

L'invention concerne des dérivés de la L-arginine de formule générale I

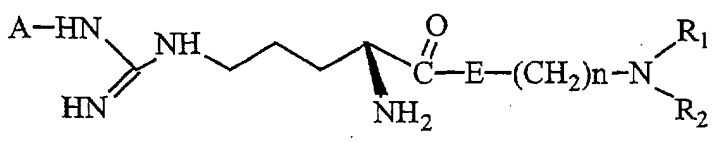

dans laquelle
A représente un atome d'hydrogène, un groupement alkyl inférieur ou le radical nitro,
E représente un atome d'oxygène ou une liaison covalente,
n est égal à un entier de 1 à 12, et
soit $R_1$ et $R_2$ représentent indépendamment une chaîne alkyl inférieur linéaire ou ramifiée,
soit $R_1$ et $R_2$ forment, ensemble, avec l'atome d'azote sur lequel ils sont rattachés, un cycle à 5 ou 6 chaînons, saturés ou insaturés, de formule

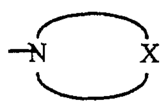

dans lequel X représente un atome d'oxygène, de soufre ou d'azote, le radical imino, alkylimino ou méthylène.

Par alkyl inférieur, on entend des radicaux alkyl comprenant de 1 à 6 atomes de carbones. Il s'agit de préférence de radicaux alkyl, linéaires ou ramifiés, comprenant de 1 à 4 atomes de carbones choisi parmi les groupes méthyl, éthyl, n-propyl, isopropyl, n-butyl, isobutyl ou *ter*-butyl.

$R_1$ et $R_2$ peuvent former, ensemble, avec l'atome d'azote sur lequel ils sont rattachés, un cycle à 5 ou 6 chaînons, saturés ou insaturés. Les cycles ainsi formés peuvent être choisis parmi le pyrrole, pipéridine, pyrrolidine, imidazole, imidazolidine, pyrazole, pyrazolidine, pyridine, pipérazine, pyrazine, pyrimidine, pyridazine, isothiazole ou morpholine.

L'invention concerne plus particulièrement les composés de formule générale I dans laquelle E représente l'atome d'oxygène, A représente le radical nitro ou l'atome d'hydrogène et $R_1$ et $R_2$ forment le cycle morpholine, pipéridine ou imidazole.

L'invention concerne également des sels pharmaceutiquement acceptable des dérivés selon l'invention. Les sels sont formés à partir d'acides organiques ou inorganiques tels que l'acide chlorhydrique, bromhydrique, acétique, fumarique, sulphonique, toluènesulphonique, maléique, carboxylique ou phosphoré. Les acides carboxyliques peuvent être choisis, par exemple, parmi les acides acétyl salicylique, salicylique, méfénamique, l'ibuprofène, le sulindac ou l'indométhacine.

Des composés de formule générale I dans laquelle dans laquelle n est égal à zéro, E représente une liaison

covalente et soit A représente l'atome d'hydrogène, $R_1$ et $R_2$ forment ensemble le cycle morpholine ou pipéridine, soit A représente le radical nitro et $R_1$ et $R_2$ forme le cycle pipéridine, ont été décrits dans la littérature ; dans ces documents (European Journal of Medicinal Chemistry, vol.23, n°6 (1988) pp 577-585; Boichemistry, vol. 23, n°1 (1984) pp 85-90; demandes de brevet FR2320088 & FR 2240720), ces dérivés ont été utilisés pour la préparation de dérivés de la L-arginine comme anti-thrombotiques. Le composé de formule I dans laquelle n est égal à zéro, E représente une liaison covalente, A représente l'atome d'hydrogène et $R_1$ et $R_2$ forment ensemble le cycle imidazole, est décrit comme intermédiaire de synthèse (Origins of Life, vol.14 (1984) pp.351-357). Cependant aucune activité pharmacologique n'a été attribuée à ces produits. L'activité d'autres dérivés de formule I, dans laquelle n est égal à zéro, E représente une liaison covalente et A représente l'atome d'hydrogène et $R_1$ et $R_2$ représentent indépendamment un radical alkyl inférieur, a été comparée avec celle de l'insuline (Z. Naturforsch. Teil C, vol.28, n°5-6, 1973; Hope-Seyler's Z. Physiol. Chem., vol.353, n°11, 1972 pp1661-1670). Mais le domaine de la thrombose et du diabète sont très différents du domaine de la NO synthase objet de la présente invention.

L'invention concerne un procédé de préparation des composés de formule générale I, procédé qui consiste à faire réagir un composé de formule générale (2)

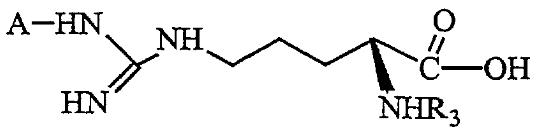

(2)

dans laquelle A représente un radical alkyl inférieur ou le radical nitro et $R_3$ un groupe protecteur, avec un composé de formule générale (3)

$$H\text{-}E\text{-}(CH_2)_n\text{-}NR_1R_2 \qquad (3)$$

dans laquelle n, E, $R_1$ et $R_2$ sont tels que définis ci-dessus, en présence d'un agent de couplage, à une température comprise entre 0 et 30°C, puis à cliver le groupe protecteur $R_3$ du composé ainsi obtenu de formule générale (4)

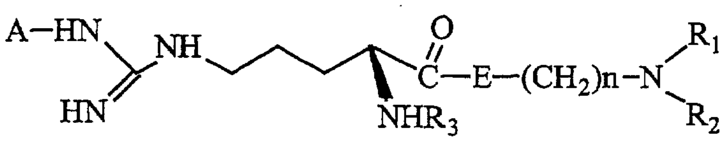

(4)

pour obtenir le composé de formule générale I dans laquelle A représente le radical nitro ou un radical alkyl inférieur. Si on désire obtenir un composé de formule générale I dans laquelle A représente un atome d'hydrogène, on utilise le composé de formule générale I correspondant, dans laquelle A représente le radical $NO_2$, et le radical nitro est clivé; le clivage peut se faire par hydrogénolyse.

Les composés de départ (3) et (2) dans lesquels A représente le radical nitro sont connus et commercialisés ; les composés (2) dans lesquels A représente un radical alkyl inférieur peuvent être préparés selon des méthodes connues (Synth. Commun., 21(1), 99(1991)). La première étape s'effectue à une température comprise entre 0 et 30°C, et de préférence entre 0°C et la température ambiante. Le solvent utilisé doit dissoudre le dérivé de la nitroarginine de formule II ; de préférence, on utilisera le DMF. La réaction est conduite en utilisant un agent de couplage. Un agent choisi parmi les agents de couplage communément utilisé en synthèse peptidique, convient ; ainsi on peut utiliser la N, N'-dicyclohexyl-carbodiimide (DCC), le benzotriazol-1-yl-oxy-tris(diméthylamino-phosphonium hexafluorophosphate (BOP) ou le benzotriazole-1-yl-oxy-tris-pyrrolidinophosphonium hexafluorophosphate (PyBOP). Des additifs peuvent également être utilisés avec l'agent de couplage comme, par exemple, le 1-hydroxybenzotriazole (HOBt) ou le 4-diméthylaminopyridine (DMAP), qui sont connus en synthèse peptidique pour accélérer les couplages, dans lesquels intervient la carbodiimide.

Le groupe protecteur $R_3$ peut être l'un quelconque des groupes protecteurs communément utilisés en synthèse peptidique, tel que benzyloxycarbonyl (Z) et t-butoxycarbonyl (Boc). Lors de la préparation d'un composé I dans lequel A représente l'atome d'hydrogène, le radical $R_3$ est de préférence un radical qui peut être clivé dans les conditions d'hydrogénolyse pour le clivage du radical nitro. De cette façon, le clivage du radical $R_3$ et du radical nitro, peuvent être conduits simultanément.

L'invention concerne aussi des compositions pharmaceutiques comprenant, à titre de principe actif, au moins un composé de formule générale I telle que définie ci-dessus.

Les compositions pharmaceutiques selon l'invention sont adaptées au mode d'administration choisi, notamment orale, parentérale. Elles peuvent donc se trouver, par exemple, sous forme de capsules, comprimés, gélules, solutions buvables, solutions injectables, ou d'une forme adaptée à la libération prolongée. Les composés selon l'invention peuvent être administrés à une dose comprise entre 0,1 et 1000 mg, par jour. De préférence, les composés peuvent être administrés à une dose comprise entre 1 et 100 mg.

L'invention concerne enfin l'utilisation des dérivés de la L-arginine de formule générale I telle que définie ci-dessus, pour la préparation d'un médicament pour le traitement de pathologies du système nerveux central et périphérique, de pathologies des dysfonctionnements du système gastrointestinal et urinaire de type inflammatoire ou non, ou de pathologies liées au système cardiovasculaire ou bronchopulmonaire.

L'invention est illustrée par les exemples suivants.

**Exemple 1 :** Morpholinoéthyl ester de la L-$N^G$-nitroarginine (L-NNA)

$A = NO_2$ $E = O$ $n = 2$ 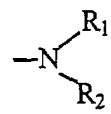 = 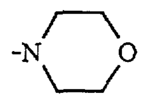

1ère étape :

Un mélange équimoléculaire ($10^{-2}$M) de L-N-Boc-$N^G$-nitroarginine et de N-β-hydroxyéthyl morpholine, est dissous dans 30 ml de diméthyl formamide (DMF) anhydre puis $3.10^{-3}$M de diméthylaminopyridine (DMAP) est ajouté. La solution est refroidit à O°C et $10^{-2}$M de dicyclohexyl carbodiimide (DDC) est ajouté. On agite 0°C pendant 10 minutes, puis on poursuit l'agitation à température ambiante pendant 24 heures. Le précipité de dicyclohexyl urée formé est filtré et le solvant éliminé. Le résidu est repris par de l'acétate d'éthyle, on lave 1 fois à l'eau, 1 fois avec NaCl saturé. L'élimination du solvant laisse un produit visqueux qui est chromatographié sur colonne de silice (éluant $CHCl_3$/MeOH9595 puis 90 : 10) ; le composé (4) est obtenu sous forme d'huile qui cristallise.
Point de fusion : 128°C
CCM : rf : 0,38 ($CHCl_3$/EtOH 85 : 15)
I.R. ($cm^{-1}$) nujol $\gamma_{NH}$ : 3400 - 3200 ; $\gamma_{ester}$ : 1740 ; $\gamma_{BOC}$ : 1710
Spectre de masse : $MH^+$ = 433
RMN-$^1$H, 100 MHz, $CDCl_3$, TMS, □δppm : 7,8 (2H $NH_2$) ; 5,5 (d, 1H, NHBoc) ; 4,3 (m, 3H, C<u>H</u> NHCO, $CO_2C\underline{H_2}$) ; 3,8 - 3,2 (m, 7H, $C\underline{H_2}NH$, $CH_2$-O-$CH_2$, NH) ; 2,5 (m, 6H, $3CH_2N$ ; 1,7 (m, 4H, $CH_2CH_2$) ; 1,4 (sing, 9H, tBu).

2ème étape: déprotection de la fonction $NH_2$

Le composé (4) obtenu dans l'étape précédente, est dissous dans du dioxane anhydre ; on rajoute goutte à goutte un excès de HCl 4N dans du dioxane à température ambiante. La solution se trouble. On laisse sous forte agitation pendant 15 heures, un précipité blanc se forme lentement. On décante le dioxane, on lave 2 fois à l'éther anhydre, on sèche au rotavapor. Le solide est dissout dans de l'eau et lyophilisé. Le composé I est obtenu sous forme de dichlorohydrate.
F : 230°C - poudre blanche hygroscopique
CCM : rf: 0,39 (2-propanol/$H_2O$/$NH_4OH$ : 7 : 3 : 0,1)
$MH^+$ (base) = 333
[$\alpha_D^{20}$] = + 3,81 ($H_2O$)
RMN-$^1$H, 100 MHz, $D_2O$, δppm :
4,5 (m, 2H, $CO_2CH_2$); 4,1 (t, 1H, C<u>H</u>($NH_2$)CO) ; 3,7(m, 4H, $CH_2OCH_2$) ; 3,4 - 3,1 (m, 8H, $CH_2N$) ; 1,9 - 1,5 (massif 4H, $CH_2$-$CH_2$).

**Exemple 2 :** Piperidinoéthyl ester de la L-NNA

A = $NO_2$ E = O n = 2 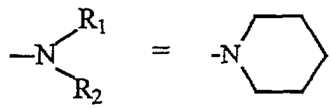

1ère étape :

Le composé correspondant de formule générale (4) est obtenu selon la méthode telle que décrite dans l'exemple 1, 1ère étape, en utilisant la N-(β-hydroxyéthyl)-pipéridine à la place de la N-(β-hydroxyéthyl)-morpholine.
Solide blanc - Point de fusion : 98°C
CCM : rf : 0,22 ($CHCl_3$/EtOH 8 : 2)
RMN-$^1$H, 100 MHz, $CDCl_3$, TMS δppm
7,6 (2H, $NH_2$) ; 5,5 (t, 1H, NHBoc) ; 4,3 (t, 3H, CH-NHCO et $CO_2CH_2$) ; 3,4 (massif, 3H, $CH_2NH$, NH) ; 2,7 - 2,3 (massif, 6H, $3CH_2N$) ; 1,7 - 1,4 (massif, 19H, tBu et $5CH_2$).

2ème étape :

Le composé I est obtenu sous forme de dichlorhydrate en suivant la méthode telle décrite dans l'exemple 1, 2ème étape.
Point de fusion : 250°C - solide blanc hygroscopique
CCM : rf: 0,36 (2-propanol/$H_2O$/$NH_4OH$: 7 : 3 : 0,1)
$MH^+$ = 331 (base)
[$\alpha_D{}^{20}$] = + 5,78 ($H_2O$)
RMN-$^1$H, 100 MHz, $D_2O$, δppm:
4,5 (m, 2H, $CO_2CH_2$) ; 4,1 (t, 1H, CH($NH_2$)CO) ; 3,5 - 2,7 (m, 8H, $4CH_2N$) ; 1,7-1,3 (m, 10H $5CH_2$).

**Exemple 3 :** 3-(diméthylamino)propyl ester de la L-NNA

A = $NO_2$ E = O n = 3 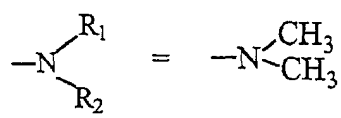

1ère étape :

Le composé correspondant de formule générale (4) est obtenu selon la méthode telle que décrite dans l'exemple 1, 1ère étape, en utilisant la 3-(diméthylamino)-propanol à la place de la N-(β-hydroxyéthyl)-morpholine. Le composé (4) est purifié par flash chromatographie ($CHCl_3$/EtOH 8 : 2). On obtient un produit visqueux.
CCM : rf: 0,17 ($CHCl_3$/MeOH 7 : 3)
RMN-$^1$H, 100 MHz, $CDCl_3$, TMS, δppm ;
5,4 (d, 1H, NHBoc) ; 4,2 (m, 3H, $COOCH_2$ et CHNHBoc) ; 3,4 (m, 2H, $NHCH_2$) ; 2,3 (m, 8H, $CH_2N$ et $(CH_3)_2N$) ; 1,7 (m, 6H, $3CH_2$).

2ème étape :

Le composé I est obtenu sous forme de dichlorhydrate en suivant la méthode telle que décrite dans l'exemple 1, 2ème étape.
Point de fusion : 178°C - poudre blanche hygroscopique
CCM : rf: 0,28 (2-propanol/$H_2O$/$NH_4OH$ : 7 : 3 : 0,1)

$MH^+$ (base) = 378
RMN-$^1H$, 100 MHz, $D_2O$, δppm :
4,15 (m, 3H, CH($NH_2$)CO, $COOCH_2$) ; 3,1 (m, 4H, $NHCH_2$ et $CH_2N$) ; 2,2 (s, 6H, $(CH_3)N$) ; 1,7 (m, 6H, $3CH_2$).

**Exemple 4 :** Morpholinopropyl ester de la L-NNA

$A = NO_2$ $E = O$ $n = 3$ 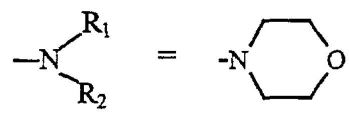

1ère étape :

En opérant tel que décrit dans l'exemple 1, 1ère étape, mais en utilisant le 3-morpholino-propanol à la place de la N-(β-hydroxyéthyl)-morpholine, le composé correspondant (4) est obtenu. Ce dernier est purifié par flash chromatographie.
Point de fusion : 106°C
CCM : rf: 0,34 ($CHCl_3$/EtOH 85 : 15)
RMN-$^1H$, 100 MHz, $CDCl_3$, TMS, δppm
7,8 (2H, $NH_2$) ; 5,4 (d, 1H, NHBoc) ; 4,3 (m, 3H, CHNHBoc, $CO_2$-$CH_2$) 3,8 - 3,3 (m, 7H, $CH_2NH$, $CH_2$-O-$CH_2$, NH) ; 2,4 (m, 6H, $3CH_2N$) ; 1,9 (m, 6H, $3CH_2$) ; 1,4 (sing, 9H, tBu).

2ème étape

Le composé I est obtenu sous forme de dichlorhydrate en suivant la méthode telle que décrite dans l'exemple 1, 2ème étape.
Point de fusion : 232°C - poudre blanche hygroscopique
CCM : rf: 0,39 (2-propanol/$H_2O$/$NH_4OH$ : 7 : 3 : 0,1)
[$\alpha_D^{20}$] = + 8,2 ($H_2O$)
MH $^+$ = 347 (base)
RMN-$^1H$, 100 MHz, $D_2O$, δppm
4,15 (m, 3H, CH($NH_2$)CO, $CO_2CH_2$) ; 3,9 - 3,3 (m, 6H, $CH_2OCH_2$, $CH_2NH$), 3,1 - 2,9 (m, 6H, $3CH_2N$) ; 2 - 1,4 (massif, 6H, $3CH_2$).

**Exemple 5 :** Morpholinohexyl ester de la L-NNA

$A = NO_2$ $E = O$ $n = 6$ 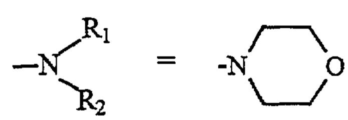

1ère étape :

En opérant tel que décrit dans l'exemple 1, 1ère étape, mais en utilisant le 6-morpholino-hexanol à la place de la N-(β-hydroxyethyl)-morpholine, le composé correspondant (4) est obtenu. Ce dernier est purifié par flash chromatographie ($CHCl_3$/EtOH 90 : 10). On obtient un produit visqueux.
Point de fusion : 99°C
CCM : rf: 0,32 ($CHCl_3$/EtOH 85 : 15)
RMN-$^1H$, 100 MHz, $CDCl_3$, TMS, δppm
7,7 (2H, $NH_2$) ; 5,4 (d, 1H, NHBoc) ; 4,25 (m, 3H, CHNHBoc, $CO_2CH_2$) 3,6 - 3,3 (m, 7H, $CH_2NH$, $CH_2OCH_2$, NH) ; 2,4 (m, 6H, $3CH_2N$); 1,8 - 1,3 (massif, 23H, tBu + $6CH_2$).

2ème étape :

Le dichlorhydrate du composé I, est obtenu selon la méthode décrite dans l'exemple 1, 2ème étape.
Point de fusion : 214°C - composé hygroscopique
CCM : rf : 0,52 (2-propanol/$H_2O$/$NH_4OH$ : 7 : 3 : 0,1)
$MH^+$ = 389 (base)
[$\alpha_D^{20}$] = + 6,67 ($H_2O$)
RMN-$^1H$, 100 MHz, $D_2O$, δppm
4,1 (m, 3H, C<u>H</u>($NH_2$)CO, $CO_2CH_2$) ; 4 -3,3 (massif, 6H, $CH_2OCH_2$, $CH_2NH$) ; 2,1 - 1,9 (m, 6H, $3CH_2N$) ; 1,8 - 1 (m, 12H, $6CH_2$).

**Exemple 6 :** Imidazoloéthyl ester de la L-NNA.

$A = NO_2$ $E = O$ $n = 2$ 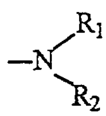 = 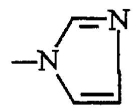

1ère étape :

Le composé (4) correspondant est obtenu selon le même mode opératoire que précédemment (Exemple 1, 1ère étape) mais en utilisant la 2-hydroxyéthyl-3-imidazole à la place de la N-(β-hydroxy-éthyl)-morpholine. Ce dernier est purifié sur colonne de silice ($CHCl_3$/EtOH 9 : puis 88 : 12).
Point de fusion : 102°C
CCM : rf: 0,19 ($CHCl_3$/EtOH/$NH_4OH$ : 85 : 15 : 0,1)
RMN-$^1H$, 100 MHz, $CDCl_3$, TMS, δppm
8 (2H, $NH_2$) ; 7,6 (1H, NH) ; 7(d, 2H, imidazole) ; 5,7 (d, 1H, NHBoc) ; 4,3 - 4,1 (m, 5H, C<u>H</u>NHBoc, $CO_2CH_2$, $CH_2$-imidazole) ; 3,3 (d, 2H, C<u>H</u>$_2$NH) ; 1,4 (m, 13H, tBu, $2CH_2$).

2ème étape :

Le composé souhaité I est obtenu sous forme de dichlorhydrate, selon la méthode précédemment décrite (exemple 1, 2ème étape)
Point de fusion : 228°C - solide jaune hygroscopique.
$MH^+$ = 314 (base)
CCM : rf: 0,39 (2-propanol/$H_2O$/$NH_4OH$: 7 : 3 : 0,1)
RMN-$^1H$, 100 MHz, $D_2O$, δppm
7,3 (d, 2H, imidazole) ; 4,4 (m, 4H, $CO_2CH_2$ et $CH_2$-imidazole); 4 (t, 1H, C<u>H</u>($NH_2$)CO) ; 3,1 (t, 2H, $CH_2NH$) ; 1,5 (m, 4H, $2CH_2$).

**Exemple 7 :** Morpholinoéthyl ester de la L-arginine

$A = H$ $E = O$ $n = 2$ 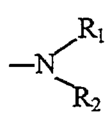 = 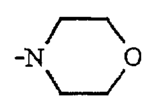

1ère étape :

En opérant selon la méthode décrite précédemment (exemple 1, 1ère étape), le composé (4) correspondant est obtenu en faisant réagir la L-N(Z)-$N^G$-nitroarginine, c'est-à-dire le composé II dans lequel le radical $R_3$ représente le groupe protecteur Z, avec la N-(β-hydroxyéthyl)-morpholine. Le composé est purifié par flash chromatographie

($CHCl_3$/EtOH 9 : 1 puis 89 : 11). On obtient un produit visqueux.
Point de fusion : 65°C
CCM : Rf: 0,28 ($CHCl_3$/EtOH : 85 : 15)
RMN-$^1$H, 100 MHz, $CDCl_3$, TMS, δppm
8,5 (1H, NH) ; 7,3 (2H, $NH_2$) ; 7,2 (s, 5H, ϕ) ; 5,7 (d, 1H, NHZ), 5 (s, 2H, $CH_2$ϕ) ; 4,2 (m, 3H, CHNHZ et $CO_2CH_2$) ; 3,6 (m, 6H, $CH_2$NH et $CH_2OCH_2$) ; 3,4 (1H, NH); 2,4 (m, 6H, $3CH_2N$) ; 1,6 (m, 4H, $2CH_2$).

2ème étape :

Le composé (4), obtenu dans l'étape précédente, est dissous dans l'éthanol et soumis à hydrogénolyse ; l'hydrogénolyse se fait dans un appareil de PARR en présence de Pd/C 10%, sous une pression de 50 psi à température ambiante. La disparition du produit de départ est suivie par CCM, la réaction est terminée en 4 heures. Après filtration, on élimine le solvant à 40°C. Le résidu visqueux est repris par le minimum d'éthanol. On ajoute de l'acide chlorhydrique 1N dans du diéthyl éther et on laisse agiter 3 heures. Un précipité se forme peu à peu. On décante et on lave le solide 2 fois avec de l'éther sec. On sèche sous pression réduite et on lyophilise. On obtient un produit légèrement jaunâtre comprenant de l'acide chlorhydrique, très hygroscopique.
Point de fusion : > 240°C (décomposition)
$MH^+$ = 288 (base)
RMN-$^1$H, 100 MHz, $D_2O$, ppm
4,4 (m, 2H, $CO_2CH_2$) ; 4,1 (t, 1H, CH($NH_2$)CO) ; 3,7 (m, 4H, $CH_2OCH_2$), 3,4 (m, 4H, $CH_2N$, $NHCH_2$) ; 3,1 (m, 4H, N$(CH_2)_2$) ; 1,6 (m, 4H, $2CH_2$).

**Exemple 8 :** Pipéridinoéthyl ester de la L-arginine

A = H    E = O    n = 2    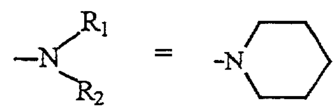

1ère étape :

Le composé correspondant de formule générale (4) peut être obtenu selon le mode opératoire de l'exemple 7, 1ère étape, mais en utilisant la N-(β-hydroxyéthyl)-pipéridine à la place de la N-(β-hydroxyéthyl)-morpholine. Le composé peut être purifié par flash chromatographie.

2ème étape :

Le chlorhydrate du composé I, peut être obtenu selon la méthode précédemment décrite (exemple 7, 2ème étape).

**Exemple 9 :** N-imidazoloéthyl ester de la L-arginine

A = H    E = O    n = 2    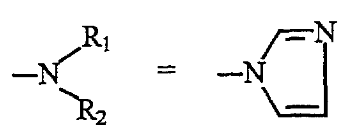

1ère étape :

Le composé correspondant de formule (4) peut être obtenu selon la méthode de l'exemple 7, 1ère étape, en utilisant la N-(β-hydroxyéthyl)-piperidine à la place de la N-(β-hydroxyéthyl)-morpholine. Le composé ainsi obtenu peut

être purifié par flash chromatographie.

<u>2ème étape</u> :

Le chlorhydrate du composé I, peut être obtenu selon la méthode précédemment décrite (exemple 7, 2ème étape).

**<u>Exemple 10</u> :** Morpholinoéthyl ester de la L-$N^G$-méthylarginine (L-NMA).

$A = CH_3$ $E = O$ $n = 2$ 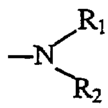 = 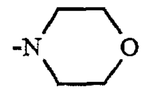

<u>1ère étape</u>

Le composé correspondant de formule générale (4) peut être obtenu selon la méthode décrite dans l'exemple 1, 1ère étape, en faisant réagir la L-N(Z)-$N^G$ méthylarginine et la N-(β-hydroxyéthyl)-morpholine. Le composé peut être purifié par chromatographie sur colonne de silice.

<u>2ème étape</u>

Le chlorhydrate du composé I peut être obtenu selon la méthode décrite dans l'exemple 7, 2ème étape.

**<u>Exemple 11</u> :** Sel d'Ibuprofen et du morpholino éthyl ester de la L-$N^G$ -nitroarginine

$2.10^{-3}$M d'ibuprofen sont dissous dans 15 ml d'eau contenant 2 équivalents d'hydroxide de sodium puis on additionne lentement cette solution à une solution aqueuse (7 ml) de $2.10^{-3}$M de morpholinoéthyl ester de la L-NNA. Le mélange se trouble, on chauffe en agitant vers 60°C pendant 30 minutes ; on obtient une solution limpide qui après refroidissement, est lyophilisée pour donner une poudre blanche.
Point de fusion>260°C - sel soluble dans l'eau.
RMN-$^1$H, 100 MHz, $D_2O$, δppm
7(4H,ϕ) ; 4,6 (m, 3H, CHNCO et $CO_2\underline{CH_2}$) ; 3,7 - 3,4 (m, 6H, $2C\underline{H_2}N$) ; 3 - 2,9 (m, 4H, $N(CH_2)_2$) ; 2,3 (d, 2H, $CH_2$- ϕ) ; 1,7 - 1,5 (massif, 5H, $2CH_2$ et CH) ; 1,2 (d, 3H, $CH_3$) ; 0,7 (d, 6H, $2CH_3$).
Le sel de l'ester de l'exemple 1 avec l'acide salicylique (exemple 12) et le sel de ce même ester avec le sulindac (exemple 13) sont obtenus selon la même méthode. Tous ces sels sont hygroscopiques.

**<u>Exemple 14</u> :** Sel de l'acide acétyl salicylique et de la morpholino amide de la L-NNA.

On dissout le morpholino-amide de la L-NNA dans 30 ml d'eau. On ajoute sous agitation l'acide acétyl salicylique, la dissolution se fait lentement. On lyophilise ensuite la solution limpide et on obtient une poudre blanche.
Point de fusion>240°C - sel soluble dans l'eau.
L'activité biologique des composés selon l'invention, est illustrée par les tests suivants.

**1- <u>Mesure de l'activité biologique *in vitro*</u>**

1.1. - <u>NO synthase consititutive endothéliale</u>

Les propriétés inhibitrices de la NO synthase endothéliale constitutive des produits sont estimées d'après leur pouvoir d'antagoniser la relaxation endothélium dépendante provoquée par le carbachol sur l'aorte isolée de rat précontractée par la phényléphrine (Auguet et al., 1992).

• <u>Matériel et Méthodes</u>

L'aorte thoracique est prélevée sur des rats mâles Sprague Dawley (290-350 g) tués par rupture cervicale.

L'aorte est placée dans du milieur Krebs Henseleit glucosé, de composition suivante : (mM), NaCl : 118 ; KCl : 4,7 ; $MgSO_4$ : 1,17 ; $KH_2PO_4$ : 1,18 ; $CaCl_2$ : 2,5 ; $NaHCO_3$ : 25 ; glucose : 11. Ce milieu est maintenu à 37°C, et continuellement traversé par un courant ce carbogène (95% $O_2$ - 5% $CO_2$). Après avoir éliminé la graisse et le tissu conjonctif en excès, les vaisseaux sont soigneusement découpés en anneaux (2 mm de large) et suspendus sous une tension de 2 g dans des cuves de 20 ml. L'enregistrement des contractions est réalisé à l'aide de capteurs isométriques reliés à un système d'acquisition de données (IOS, Dei-Lierre).

• Produits et traitement

Après une heure de repos, le tonus de l'artère est augmenté par la phényléphrine (PE $10^{-6}$ M). Lorsque la contraction a atteint son maximum (10 minutes), le carbachol ($10^{-5}$ M) est introduit dans la cuve pour vérifier l'intégrité de la couche endothéliale.
Les préparations sont ensuite lavées et après 45 minutes de repos, la PE ($10^{-6}$M) est réintroduite dans le bain au maximum de la contraction, le carbachol $10^{-5}$M est injecté lorsque le maximum de la relaxation est atteint, les produits testés sont introduits dans le bain en doses cumulées.

Les résultats sont résumés dans le tableau 1 suivant :

TABLEAU 1

| PRODUITS | $CI_{50}$ (μM) |
|---|---|
| L-NMMA | 12 |
| L-NA | 7 |
| Aminoguanidine | > 100 |
| Exemple 1 | 10 |
| Exemple 2 | 30 |
| Exemple 3 | 75 |
| Exemple 4 | 10 |
| Exemple 5 | 5 |
| Exemple 6 | 80 |
| Exemple 7 | > 100 |
| Exemple 11 | 5 |
| Exemple 12 | 10 |
| Exemple 13 | 9 |

1.2. - NO synthase constitutive neuronale

• Introduction

La mesure de l'activité de la NO synthase a été effectuée selon le protocole décrit par Bredt et Snyder (1990).

• Matériel et méthodes

Les cervelets de rat Sprague Dawley (280 g, Charles River) sont prélevés rapidement, disséqués à 4°C et homogénéisés dans un volume de tampon d'extraction correspondant à 5 ml de tampon HEPES/g de tissu à l'aide de potter de Thomas (10 aller/retours). Les homogénats sont ensuite centrifugés (21000 g pendant 15 minutes à 4°C). Le surnageant est prélevé et passé sur une colonne de 1 ml de Dowex AG 50 WX-8, $Na^+$ (forme (BioRad) préalablement rincée avec de l'eau distillée et du tampon d'extraction. Après passage sur colonne, les échantillons sont conservés à 4°C et dosés rapidement.

Le dosage se fait dans des tubes à essai en verre dans lesquels sont distribués 100 μl de tampon d'incubation contenant 100 mM d'HEPES, pH 7,4 2 mM d'EDTA, 2,5 mM de $CaCl_2$, 2 mM de dithiotréitol, 2 mM de NADPH réduit, 10 μg/ml de calmoduline et 10 μM de tétrahydrobioptérine. On ajoute 25 μl d'une solution contenant 100 nM d'arginine tritiée (activité spécifique : 56,4 Ci/mmole, Amersham) et 40 μM d'arginine non radioactive. La réaction est initiée en ajoutant 50 μl d'homogénat, le volume final étant 200 μl (les 25 μl manquants sont soit de l'eau, soit un produit testé). Après 30 minutes d'incubation à température ambiante, la réaction enzymatique est arrêtée par addition de 2 ml de tampon d'arrêt (20 mM d'HEPES, pH 5,5 ; 2 mM EDTA). Les échantillons sont passés sur des colonnes de 1 ml de résine Dowex AG 50 WX-8, $Na^+$ forme et élués avec 2 ml d'eau distillée. Après addition de 10 ml de liquide à scintillation,

la radioactivité est quantifiée à l'aide d'un spectomètre à scintillation liquide.
Les blancs sont réalisés selon le même protocole expérimental, les 50 µl d'échantillon étant remplacés par 50 µl de tampon d'extraction. La radioactivité totale est déterminée pour chaque essai.

Les résultats sont exprimés en pmoles de citrulline formées/min/mg de protéines. La concentration en protéine se fait selon la méthode de Bradford (1976) (tableau 2).

TABLEAU 2

| PRODUITS | $CI_{50}$ (µM) |
|---|---|
| L-NMMA | 2,8 |
| L-NA | 0,6 |
| Aminoguanidine | 510 |
| Exemple 1 | 2,3 |
| Exemple 2 | 4 |
| Exemple 3 | 10 |
| Exemple 4 | 1,5 |
| Exemple 5 | 2 |
| Exemple 6 | 1,8 |
| Exemple 7 | 10 |
| Exemple 11 | 0,36 |
| Exemple 12 | 1 |
| Exemple 13 | 0,7 |

1.3. - NO synthase inductible

Les macrophages produisent du monoxyde d'azote à partir d'une NO synthase inductible par des stimuli proinflammatoires. La NO synthase inductible de macrophage est préparée à partir d'un homogénat de cellules myélomonocytaire $J774A_1$ préalablement stimulé pendant 48 h par du LPS (E. Coli) et de l'$IFN_{\gamma}$.

• Matériel et Méthode

1. - Cellules : Culture et induction de la NO synthase

Les cellules $J774A_1$ (ATCC réf. TIB 67) sont cultivées en DMEM à 10% de SVF à 37°C sous une atmosphère à 5% de $CO^2$. Elles sont ensemencées à raison de 5 x $10^3$ cellules/$cm^2$ dans des flacons de 150 $cm^2$. Les incubations se font en présence de LPS (1µg/ml) et d'IFN-γ murin (50 U/ml) dans du DMEM à 10% de SVF pendant 24 heures.

2. - Préparation de la NO synthase partiellement purifiée

Les cellules sont lavées au PBS puis grattées au Cell Scraper dans 4 ml de tampon A froid (4°C) (tampon A : hepes 50 mM, dithiothréitol 0,5 mM ajusté à pH 4 avec NaOH 1N). On ajoute extemporanément : pepstatine A 1 mg/ml, leupeptine 1 mg/ml, inhibiteur de trypsine du soja 1 mg/ml, antipaïne 1 mg/ml, PMSF (Pefobloc) 10 mg/ml. Après centrifugation (1000 g, 4°C, 5 minutes), les culots sont rassemblés et repris dans 1 ml de tampon A puis soniqués à 4°C et l'homogénat subit une ultra-centrifugation (100.000 g, 4°C, 60 minutes). Au surnageant est ajouté 10% de glycérol avant de la congeler à -80°C, en l'ayant préalablement aliquoté. La préparation est testée le jour même, mais elle garde une activité après 10 jours de conservation à -80°C. Un aliquot est gardé pour le dosage des protéines par la microméthode de Bradford.

3 - Test enzymatique

Le test consiste en la transformation par la NO synthase de la L-arginine en L-citrulline.

a. - Conversion de la L-arginine en L-citrulline

- Tampon réactionnel ou tampon B : Hepes 100 mM, Dithiothréitol 1 mM, ajusté à pH 7,4 avec NaOH 1N. Ce tampon se garde quelques jours à 4°C. Extemporanément, on ajoute les cofacteurs de la NO synthase soit : Tétrahydrobioptérine 10 µM, FAD 10 µM, NADPH 2 mM, $CaCl_2$ 2,5 mM, BSA 1 mg/ml (afin de solubiliser l'enzyme).
- Solution de L-arginine :

  La L-arginine est à 40 µM final. Pour suivre la réaction, nous pratiquons une dilution isotopique en ajoutant à la solution de la ($^3$H)-L-arginine en concentration finale de 100 nM.

- Préparation enzymatique :

  Selon un test fait préalablement donnant l'activité enzymatique, la solution est utilisée pure ou au 1/10°.

- Inhibiteur :

  Ils sont préparés dans le tampon B complet à partir d'une solution concentrée en milieu aqueux ou en DMSO et testés vis-à-vis d'un témoin sans inhibiteur. Un témoin DMSO est ajouté le cas échéant.

| Volumes (µl) utilisés dans le test | | | | |
|---|---|---|---|---|
| Echantillon | Tampon B complet | Solution de L arginine | Préparation enzymatique | Tampon A complet |
| Blanc | 125 | 25 | - | 50 |
| Témoin | 125 | 25 | 50 | - |
| Inhibiteur | 125 | 25 | 50 | - |

L'incubation se fait au bain- marie à 37°C pendant 15 minutes.

- Arrêt de la réaction :

L'arrêt de la réaction se fait par 2 ml de tampon C : Hepes 20 mM, EDTA 2 mM, pH 5,5 ajusté avec HCl 1N

- Séparation de la L-arginine et de la L-citrulline:

Celle-ci s'effectue sur 0,5 ml de Dowex 50X-8 (résine échangeuse de cation) préalablement équilibré dans le tampon C, dans des séringues de 2 ml bouchées par une bille de verre ne laissant ainsi s'écouler que le liquide.

La 5OX-8 retient la L-arginine. On effectue le dépôt de l'échantillon que l'on recueille puis on élue le reste de la citrulline par 2 ml d'eau distillée.

Aux 4 ml de milieu aqueux sont ajoutés 16 ml d'Instagel Plus et on compte les fioles à scintillation dans un capteur Packard.

- Calculs :

On effectue pour chaque valeur le calcul suivant :

$$\frac{\text{cpm (échantillon) - cpm (blanc)}}{\text{cpm (témoin) - cpm (blanc)}}$$

On fait ensuite la droite de régression sous Fig.P 6C et on détermine la $CI_{50}$ des produits. les $CI_{50}$ sont la moyenne de deux expériences pratiquées sur des préparations enzymatiques différentes. Les résultats sont présentés dans le tableau 3.

TABLEAU 3

| PRODUITS | $CI_{50}$ (μM) |
|---|---|
| L-NA | 20,5 |
| L-NMMA | 9,5 |
| Aminoguanidine | 29,6 |
| Exemple 1 | 29 |
| Exemple 2 | 53 |
| Exemple 3 | 30 |
| Exemple 4 | 110 |
| Exemple 5 | 380 |
| Exemple 6 | 15 |
| Exemple 7 | 22 |
| Exemple 11 | 40 |
| Exemple 12 | 28 |
| Exemple 13 | 38 |

**2- Mesure de l'activité biologique *in vivo***

Oedème à la carragénine

• Principe

L'injection sous plantaire d'une suspension de carragénine, mucopolysaccharides extraits d'algues, induit une réaction inflammatoire locale. Les composés anti-inflammatoires sont capables d'antagoniser cet oedème de façon différentielle selon leur mode d'action (Winter et al., 1962). Les inhibiteurs de NO synthase ont montré un certain effet sur ce test (Antunes et al., 1991).

• Matériel et Méthodes

Des lots de 8 rats mâles Sprague Dawley VAF (Charles River, St Aubin les Elbeuf) de 140 à 215 g à jeun depuis 18 heures ont été utilisés (n = 8).

- l'oedème est obtenu par injection dans le coussinet plantaire d'une des pattes postérieures, d'une suspension de carragénine à 1 % en sérum physiologique à raison de 0,1 ml par animal.
- le volume de la patte est mesuré par pléthysmographie avant, puis 1h30 et 3 heures après injection de carragénine. Un pourcentage d'inflammation de la patte est calculé pour chaque animal et chaque temps de mesure (pléthysmomètre à eau - Ugo Basile).

• Résultats

Les résultats sont présentés dans le tableau 4. (NS : non significatif, * : significatif, ** : très significatif, *** : hautement significatif).

**TABLEAU 4**

| PRODUITS | Dose (mg/kg) | % Diminution de l'inflammation 90 min | 180 min |
|---|---|---|---|
| L-NA | 15 | - 60,2 ** | - 52,8 ** |
| L-NMMA | 15 | - 30,5* | - 12,9 NS |
| Aminoguanidine | 15 | - 9 NS | - 13 NS |
| Exemple 1 | 15 | - 62,5 ** | - 55,7 ** |
| Exemple 2 | 15 | - 58,3 ** | - 56,3 ** |
| Exemple 3 | 15 | - 48,7** | - 61,2 ** |
| Exemple 5 | 15 | - 75,2 ** | - 48 ** |
| Exemple 11 | 15 | - 85,9 ** | - 85 *** |

**Revendications**

1. Dérivés de la L-arginine de formule générale I

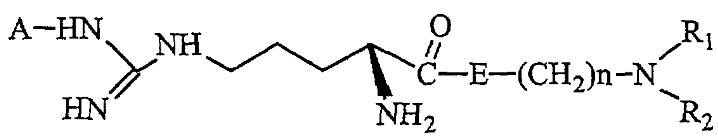

I

dans laquelle
A représente un atome d'hydrogène, un groupement alkyl de 1 à 6 atomes de carbone ou le radical nitro,
E représente un atome d'oxygène ou une liaison covalente,
n représente un entier de 1 à 12, et
soit $R_1$ et $R_2$ représentent indépendamment une chaîne alkyl linéaire ou ramifiée soit $R_1$ et $R_2$ forment, ensemble, avec l'atome d'azote sur lequel ils sont rattachés, un cycle à 5 ou 6 chaînons, saturés ou insaturés, de formule

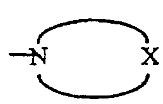

dans lequel X représente un atome d'oxygène, de soufre ou d'azote, le radical imino, alkylimino ou méthylène ; et leurs sels pharmaceutiquement acceptables.

2. Dérivés selon la revendication 1, pour lesquels E représente l'atome d'oxygène, A représente le radical nitro ou l'atome d'hydrogène et $R_1$ et $R_2$ forment le cycle morpholine, pipéridine ou imidazole.

3. Procédé de préparation des composés selon l'une des revendications 1 à 2, procédé qui consiste à faire réagir un composé de formule générale (2)

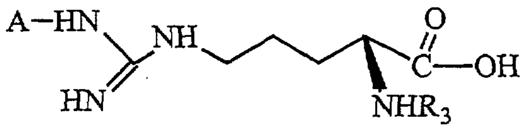

(2)

dans laquelle A représente un radical alkyl de 1 à 6 atomes de carbone ou le radical nitro et $R_3$ un groupe protecteur, avec un composé de formule générale (3)

$$H\text{-}E\text{-}(CH_2)_n\text{-}NR_1R_2 \quad (3)$$

dans laquelle n, E, $R_1$ et $R_2$ sont tels que définis à la revendication 1, en présence d'un agent de couplage, à une température comprise entre 0 et 30°C, puis à cliver le groupe protecteur $R_3$ du composé ainsi obtenu de formule générale (4)

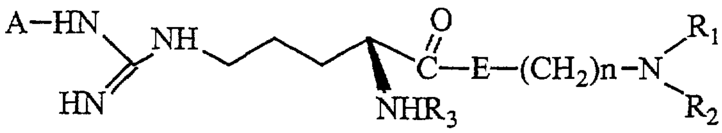

(4)

afin d'obtenir le composé de formule générale I dans laquelle A représente le radical nitro ou un radical alkyl de 1 à 6 atomes de carbone et, si le composé de formule générale I dans laquelle A représente un atome d'hydrogène est souhaité, à déprotéger le composé de formule générale I correspondant, dans laquelle A représente le radical $NO_2$, par clivage du radical nitro.

**4.** Procédé selon la revendication 3, dans lequel l'agent de couplage utilisé est la N, N'-dicyclohexyl-carbodiimide (DCC), le benzotriazol-1-yl-oxy-tris(diméthyl-amino-phosphonium hexafluorophosphate (BOP) ou le benzotriazole-1-yl-oxy-tris-pyrrolidinophosphonium hexafluorophosphate (PyBOP).

**5.** Procédé selon l'une des revendications 3 à 4, dans lequel le 1-hydroxybenzotriazole (HOBt) ou le 4-diméthylaminopyridine (DMAP) est utilisé comme additif de l'agent de couplage.

**6.** Procédé selon l'une quelconque des revendications 3 à 5, dans lequel le groupe protecteur $R_3$ est le benzyloxycarbonyl (Z) et t-butoxycarbonyl (Boc).

**7.** Procédé selon l'une quelconque des revendications 3 à 6, pour la préparation d'un composé I dans lequel A représente l'atome d'hydrogène, dans lequel le radical $R_3$ et le radical nitro sont clivés simultanément.

**8.** Compositions pharmaceutiques comprenant, à titre de principe actif, au moins un composé selon l'une des revendications 1 à 2, en association avec au moins un diluent ou support pharmaceutiquement acceptable.

**9.** Utilisation des dérivés de la L-arginine tels que définis à la revendication 1, pour la préparation d'un médicament pour le traitement de pathologies du système nerveux central et périphérique.

**10.** Utilisation des dérivés de la L-arginine tels que définis à la revendication 1, pour la préparation d'un médicament pour le traitement de pathologies des dysfonctionnements du système gastrointestinal et urinaire de type inflammatoire ou non.

**11.** Utilisation des dérivés de la L-arginine tels que définis à la revendication 1, pour la préparation d'un médicament pour le traitement de pathologies liées au système cardiovasculaire ou bronchopulmonaire.

## Claims

1. Derivatives of L-arginine of general formula I

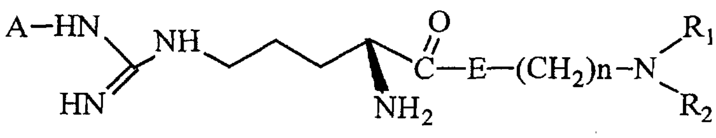

I

in which :

A represents a hydrogen atom, an alkyl group of 1 to 6 carbon atoms, or the nitro radical,
E represents an oxygen atom or a covalent bond ;
n represents an integer from 1 to 12 ; and
either $R_1$ and $R_2$ represent independently a branched or linear alkyl chain,
or $R_1$ and $R_2$ form together with the nitrogen atom to which they are attached, a ring of 5 or 6 groups, saturated or unsaturated, of the formula :

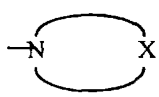

in which X represents an atom of oxygen, sulfur or nitrogen, the imino, alkylimino, or methylene radical ; and their pharmaceutical acceptable salts.

2. Derivatives according to claim 1 for which E represents the oxygen atom, A represents the nitro radical or a hydrogen atom, and $R_1$ and $R_2$ form the morpholine, piperidine, or imidazole ring.

3. A process for preparing compounds according to any of the claims 1 to 2, which process consists in reacting a compound of general formula (2) :

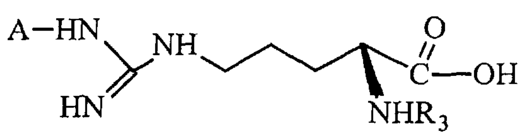

(2)

in which A represents an alkyl radical of 1 to 6 carbon atoms or the nitro radical and $R_3$ a protecting group, with a compound of general formula (3) :

$$H\text{-}E\text{-}(CH_2)_n\text{-}NR_1R_2 \qquad (3)$$

in which n, E, $R_1$ and $R_2$ are as defined above, in the presence of a coupling agent, at a temperature comprising between 0 and 30° C, then to cleave the protecting group $R_3$ of the compound thus obtained from the general formula (4):

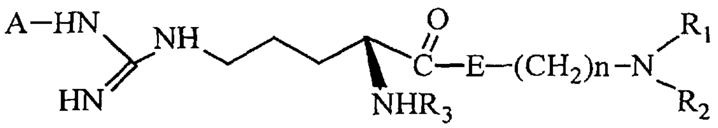

(4)

in order to obtain the compound of general formula I in which A represents the nitro radical or an alkyl radical of 1 to 6 carbon atoms and, if the compound of general formula I in which A represents a hydrogen atom is desired, to de-protect the corresponding compound of general formula I in which A represents the $NO_2$ radical by cleavage of the nitrogen radical.

**4.** A process according to claim 3 in which the coupling agent used is N,N'-dicyclohexyl carbodiimide (DCC), benzotriazol-1-yl-oxy-tris(dimethyl-amino)-phosphonium hexafluorophosphate (BOP) or benzotriazole-1-yl-oxy-tris-pyrrolidinophosphonium hexafluorophosphate (PyBOP).

**5.** A process according to any of the claims 3 to 4 in which 1-hydroxybenzotriazole (HOBt) or 4-dimethylaminopyridine (DMAP) is used as an additive to the coupling agent.

**6.** A process according to any of the claims 3 to 6 in which the $R_3$ protecting group is benzyloxycarbonyl (Z) and t-butoxycarbonyl (Boc).

**7.** A process according to any of the claims 3 to 6, for the preparation of a compound I in which A represents a hydrogen atom, in which the radical $R_3$ and the nitro radical are cleaved simultaneously.

**8.** Pharmaceutical compositions containing, as active principle, at least one compound according to any of the claims 1 to 2, in association with at least one pharmaceutically acceptable support or diluent.

**9.** Use of derivatives of L-arginine as defined in claim 1, for the preparation of a medicament for the treatment of pathologies of central or peripheral nervous system.

**10.** Use of derivatives of L-arginine as defined in claim 1, for the preparation of a medicament for the treatment of pathologies of dysfunctioning of the gastrointestinal and urinary system, of inflammatory type or not.

**11.** Use of derivatives of L-arginine as defined in claim 1, for the preparation of a medicament for the treatment of pathologies connected with the cardiovascular or bronchopulmonary system.

**Patentansprüche**

**1.** L-Arginin-Derivate der allgemeinen Formel I

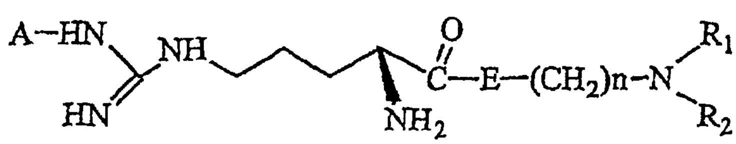

I

in der
A ein Wasserstoffatom, eine Alkylgruppe mit 1 bis 6 Kohlenstoffatomen oder einen Nitrorest darstellt,
E ein Sauerstoffatom oder eine kovalente Bindung darstellt, n eine ganze Zahl von 1 bis 12 darstellt und
entweder $R_1$ und $R_2$ unabhängig voneinander eine verzweigte oder unverzweigte Alkylkette darstellen
oder $R_1$ und $R_2$ zusammen mit dem Stickstoffatom, an dem sie gebunden sind, einen gesättigten oder ungesättigten Ring mit 5 oder 6 Gliedern der Formel

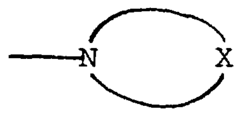

bilden, in der X ein Sauerstoff-, Schwefel- oder Stickstoffatom, einen Imino-, Alkylimino- oder Methylenrest darstellt; und ihre pharmazeutisch verträglichen Salze.

**2.** Derivate nach Anspruch 1, bei denen E ein Sauerstoffatom darstellt, A einen Nitrorest oder ein Wasserstoffatom darstellt und $R_1$ und $R_2$ einen Morpholin-, Piperidin- oder Imidazolring bilden.

**3.** Verfahren zur Herstellung der Verbindungen nach einem der Ansprüche 1 bis 2, das darin besteht, dass man eine Verbindung der allgemeinen Formel (2)

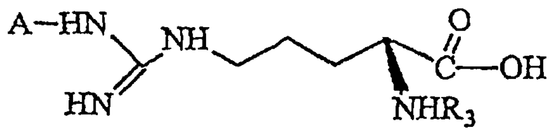
(2)

in der A einen Alkylrest mit 1 bis 6 Kohlenstoffatomen oder einen Nitrorest und $R_3$ eine Schutzgruppe darstellt, mit einer Verbindung der allgemeinen Formel (3)

$$H\text{-}E\text{-}(CH_2)_n\text{-}NR_1R_2 \quad (3)$$

in der n, E, $R_1$ und $R_2$ die in Anspruch 1 angegebene Bedeutung haben, in Gegenwart eines Kupplungsmittels bei einer Temperatur zwischen 0 und 30°C reagieren lässt, dann die Schutzgruppe $R_3$ der auf diese Weise erhaltenen Verbindung der allgemeinen Formel (4)

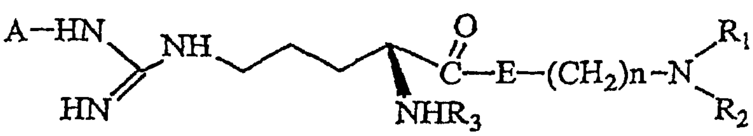
(4)

abspaltet, um die Verbindung der allgemeinen Formel I zu erhalten, in der A einen Nitrorest oder einen Alkylrest mit 1 bis 6 Kohlenstoffatomen darstellt, und, wenn die Verbindung der allgemeinen Formel I, in der A ein Wassestoffatom darstellt, gewünscht wird, die entsprechende Verbindung der allgemeinen Formel I, in der A einen $NO_2$-Rest darstellt, durch Abspalten des Nitrorestes entschützt.

**4.** Verfahren nach Anspruch 3, in dem das verwendete Kupplungsmittel N,N'-Dicyclohexylcarbodiimid (DCC), Benzotriazol-1-yl-oxy-tris(dimethylamino)phosphoniumhexafluorphosphat (BOP) oder Benzotriazol-1-yl-oxy-tris-pyrrolidinophosphoniumhexafluorphosphat (PyBOP) ist.

**5.** Verfahren nach einem der Ansprüche 3 bis 4, bei dem 1-Hydroxybenzotriazol (HOBt) oder 4-Dimethylaminopyridin (DMAP) als Additiv zu dem Kupplungsmittel verwendet wird.

**6.** Verfahren nach einem der Ansprüche 3 bis 5, bei dem die Schutzgruppe $R_3$ Benzyloxycarbonyl (Z) und t-Butoxycarbonyl (Boc) ist.

**7.** Verfahren nach einem der Ansprüche 3 bis 6 zur Herstellung einer Verbindung I, in der A ein Wasserstoffatom darstellt, in dem der Rest $R_3$ und der Nitrorest gleichzeitig abgespalten werden.

8. Pharmazeutische Zusammensetzungen, die als Wirkstoff mindestens eine Verbindung nach einem der Ansprüche 1 bis 2 in Kombination mit mindestens einem pharmazeutisch verträglichen Verdünnungsmittel oder Träger besitzen.

9. Verwendung der L-Arginin-Derivate nach Anspruch 1 für die Herstellung eines Medikaments zur Behandlung von Erkrankungen des Zentralnervensystems und des peripheren Nervensystems.

10. Verwendung der L-Arginin-Derivate nach Anspruch 1 für die Herstellung eines Medikaments zur Behandlung von Erkrankungen im Zusammenhang mit entzündlichen oder nicht entzündlichen Funktionsstörungen des Magendarm- und Harnsystems.

11. Verwendung der L-Arginin-Derivate nach Anspruch 1 für die Herstellung eines Medikaments zur Behandlung von mit dem kardiovaskulären oder bronchopulmonären System verbundenen Erkrankungen.